Europäisches Patentamt

European Patent Office

Office européen des brevets

(19)

(11) **EP 0 758 221 B1**

(12) **EUROPÄISCHE PATENTSCHRIFT**

(45) Veröffentlichungstag und Bekanntmachung des Hinweises auf die Patenterteilung:
**03.07.2002 Patentblatt 2002/27**

(51) Int Cl.⁷: **A61K 7/06**

(21) Anmeldenummer: **95917328.7**

(86) Internationale Anmeldenummer:
**PCT/EP95/01437**

(22) Anmeldetag: **18.04.1995**

(87) Internationale Veröffentlichungsnummer:
**WO 95/28908 (02.11.1995 Gazette 1995/47)**

(54) **HAARBEHANDLUNGSMITTEL**

HAIR-TREATMENT AGENTS

AGENTS DE TRAITEMENT CAPILLAIRE

(84) Benannte Vertragsstaaten:
**AT BE CH DE DK ES FR GB GR IT LI NL PT SE**

(30) Priorität: **26.04.1994 DE 4414424**

(43) Veröffentlichungstag der Anmeldung:
**19.02.1997 Patentblatt 1997/08**

(73) Patentinhaber: **Henkel Kommanditgesellschaft auf Aktien
40191 Düsseldorf (DE)**

(72) Erfinder:
• **EMMERLING, Winfried
D-41469 Neuss (DE)**
• **HOFMANN, Hans-Peter
D-40599 Düsseldorf (DE)**
• **KADE, Rainer
D-42651 Solingen (DE)**
• **SCHIEFERSTEIN, Ludwig
D-40882 Ratingen (DE)**

(56) Entgegenhaltungen:
| | |
|---|---|
| **EP-A- 0 590 604** | **WO-A-91/15186** |
| **WO-A-91/15187** | **WO-A-92/21319** |
| **WO-A-93/03703** | **WO-A-94/01079** |
| **WO-A-94/02112** | **FR-A- 2 122 074** |
| **GB-A- 851 773** | **US-A- 5 059 414** |

**Beschreibung**

Gegenstand der Erfindung ist die Verwendung eines unlöslichen Polymers mit einer Glasübergangstemperatur zwischen -20 und + 70°C, bestehend im wesentlichen aus Monomeren, ausgewählt aus der Gruppe, die Ester der Acrylsäure, Ester der Methacrylsäure und Styrol umfaßt in Haarbehandlungsmitteln, zur Behandlung von Haaren mit der Maßgabe, daß die Haarbehandlungsmittel, enthaltend übliche kosmetische Bestandteile, in Form einer wäßrigen Dispersion dieses Polymeren vorliegen, weiterhin ein wasserlösliches Polymer enthalten und Wasser als einziges Lösungsmittel enthalten.

Haarbehandlungsmittel in Form wäßriger Lösungen oder Emulsionen von Polymeren mit Acrylatmonomeren sind beispielsweise aus den internationalen Patentanmeldungen WO 94/02112 und WO/01079 sowie der europäischen Patentanmeldung 0 590 604 bekannt. Wäßrige Haarbehandlungsmittel, die wasserunlösliche Polymere sowie für diese geeignete wasserunlösliche Lösungsmittel enthalten, sind aus der internationalen Patentanmeldung WO 92/21319 bekannt. Die internationalen Patentanmeldungen WO 91/15187 und WO 91/15186 offenbaren wäßrige Haarbehandlungsmittel, die Polymere mit bestimmten Löslichkeitsparametern sowie nichtwäßrige Lösungsmittel zur Solubilisierung dieser Polymere enthalten.

Weiterhin bekannt aus der deutschen Offenlegungsschrift 42 24 761 ist die Verwendung wasserlöslicher oder wasserdispergierbarer Polykondensate mit Glastemperaturen oberhalb von +20 °C in Haarbehandlungsmitteln.

Schließlich sind aus der Publikation CB-14B der Firma Eastman Chemical Company Haarsprays in Form wasserbasierter Polyesterdispersionen bekannt. Diese haben den Nachteil, daß bei hohen Polymerkonzentrationen häufig bereits nach kurzer Zeit die Düsen der Applikationsbehälter, insbesondere bei Pumpsprays, verstopfen. Daher können diese Polymeren bei Konsumentenprodukten nur in relativ geringen Konzentrationen eingesetzt werden, so daß beim Aufbringen der benötigten Polymermengen auf das Haar gleichzeitig eine große Wassermenge auf das Haar aufgebracht wird.

Die genannten Druckschriften liefern aber keinerlei Hinweise auf die besonders vorteilhaften Eigenschaften der erfindungsgemäßen Mittel.

Die erfindungsgemäß verwendeten Haarbehandlungsmittel liegen in Form wäßriger Dispersionen vor und enthalten ein unlösliches Polymeres.

Unter wäßrigen Dispersionen im Sinne der Erfindung sind solche Dispersionen zu verstehen, deren äußere Phase überwiegend aus Wasser besteht, wobei Wasser das einzige Lösungsmittel ist.

Ein Polymer ist dann unlöslich im Sinne der Erfindung, wenn es bei Raumtemperatur in Wasser zu weniger als 1 Gew.-% löslich ist.

Die erfindungsgemäß eingesetzten wasserunlöslichen Polymeren weisen Glasübergangstemperaturen zwischen -20 °C und +70 °C auf. Polymeren mit Glasübergangstemperaturen zwischen -10 °C und +50 °C, insbesondere zwischen +10 °C und +30 °C, sind bevorzugt.

Die erfindungsgemäß eingesetzten wasserunlöslichen Polymeren bestehen im wesentlichen, d. h. zu mindestens 80 Gew.-%, bezogen auf das Polymere, aus Estern der Acrylsäure und/oder Methacrylsäure und/oder Styrol.

Als Ester der Acryl- bzw. Methacrylsäure kommen alle bekannten Ester dieser Säuren mit linearen und verzweigten, gesättigten und ungesättigten aliphatischen Alkoholen, aromatischen Alkoholen und aliphatisch-aromatischen Alkoholen in Betracht. Bevorzugt sind Ester mit linearen und verzweigten, gesättigten und ungesättigten aliphatischen Alkoholen mit 1 bis 22 Kohlenstoffatomen. Solche Alkohole sind beispielsweise Methanol, Ethanol, n- und iso-Propanol, n- und iso-Butanol-1, n-Pentanol-1, n-Hexanol-1, n-Heptanol-1, n-Octanol-1, n-Octanol-2, n-Decanol-1, n-Dodecanol-1 (Laurylalkohol), Myristylalkohol, Cetylalkohol, Stearylalkohol, Behenylalkohol, Oleylalkohol und Linoleylalkohol. Besonders bevorzugt sind Alkohole mit 1 bis 6, insbesondere mit 1 bis 4, Kohlenstoffatomen.

Erfindungsgemäß ebenfalls verwendbar, wenngleich weniger bevorzugt, sind die Ester der Acryl- und Methcrylsäure mit den entsprechenden alkoxylierten, insbesondere ethoxylierten Alkoholen. Dabei können Alkoxylierungsgrade von 1 bis etwa 10 vorliegen.

Styrole im Sinne der Anmeldung sind auch Verbindungen, die eine kurzkettige Alkylgruppe, insbesondere eine Methylgruppe, am α-C-Atom der Vinylgruppe aufweisen. Der Gehalt an Styrolgruppen im Polymer wird bevorzugt auf weniger als 70 Gew.-%, bezogen auf das Polymere, begrenzt, da es sonst unter Umständen schwierig ist, Polymere mit Glastemperaturen in dem genannten Bereich zu erhalten.

Es ist ein besonderer Vorteil, daß erfindungsgemäß Polymere eingesetzt werden können, die nur aus wenigen Monomeren bestehen. So weisen Mittel mit Homopolymeren oder Copolymeren aus nur zwei Monomeren überraschend gute Eigenschaften aus. Es kann daher bevorzugt sein, im Rahmen der erfindungsgemäßen Lehre solche Homo- oder Copolymeren einzusetzen.

Die wasserunlöslichen Polymere enthalten daher auch bevorzugt neben den Acrylsäureestern, Methacrylsäureestern und Styrolen keine weiteren Bausteine, wobei Verunreinigungen der Monomeren und Verbindungen, die den Kettenabbruch bewirken, nicht als weitere Bausteine angesehen werden.

Es hat sich jedoch gezeigt, daß auch Polymere, die bis zu 20 Gew.-%, insbesondere bis zu 10 Gew.-%, an weiteren Bausteinen aufweisen, erfindungsgemäß verwendet werden können. Solche weiteren Bausteine können sein:

- Acrylamide und Methacrylamide,
- Acrylsäure, Methacrylsäure und deren Alkalimetall-, Erdalkalimetall-, Aluminium-, Ammonium- und Alkylolammoniumsalze,
- Crotonsäure, deren Ester und Alkalimetall-, Erdalkalimetall-, Aluminium-, Ammonium- und Alkylolammoniumsalze,
- Vinyl- und Allylverbindungen,
- aminogruppenhaltige Monomere.

Dabei traten die erfindungsgemäßen Vorteile in der Regel nur bei solchen Polymeren auf, bei denen der jeweilige Anteil der weiteren Bausteine auf weniger als 5 Gew.-%, bezogen auf das Polymer, begrenzt war. Ebenso hat sich gezeigt, daß es bei Einbeziehung ionischer Bausteine vorteilhaft ist, wenn entweder nur kationische oder nur anionische Bausteine im Polymer vertreten waren. Diese Polymeren wiesen deutlich bessere Eigenschaften auf als solche, in denen sowohl anionische als auch kationische Bausteine vertreten waren.

Bei der Auswahl der Monomeren und der fakultativen weiteren Bausteine ist unbedingt darauf zu achten, daß die Glastemperatur im Bereich zwischen -20 °C und +70 °C liegt. Hier kann der Fachmann aufgrund der bekannten Gesetzmäßigkeiten, die zwischen den Glastemperaturen der jeweiligen Homopolymeren und den entsprechenden Copolymeren, zwanglos die notwendige Vorauswahl treffen. Bei Polymeren mit weiteren, ionischen, Bausteinen ist unbedingt darauf zu achten, daß die Löslichkeit nicht zu stark zunimmt, um die damit verbundenen Nachteile zu vermeiden.

Die mittleren Molmassen der erfindungsgemäß verwendeten Polymeren liegen in der Regel zwischen 20.000 und 2.000.000; Werte zwischen 50.000 und 500.000 haben sich als besonders vorteilhaft erwiesen. Dem Fachmann sind die Möglichkeiten, bei der Polymersynthese Molmassen in einem gewünschten Bereich zu erhalten, bekannt.

Die erfindungsgemäß verwendeten Haarbehandlungsmittel enthalten das unlösliche Polymere, in Abhängigkeit vom Typ des Haarbehandlungsmittels, der keinen Einschränkungen unterliegt, bevorzugt in Mengen von 0,1 bis 30 Gew.-%, insbesondere 1 bis 20 Gew.-%, bezogen auf das gesamte Mittel.

Selbst bei höheren Konzentrationen, d.h. bei Konzentrationen oberhalb von etwa 10 %, zeichnen sich die erfindungsgemäßen Mittel im Vergleich zu Mitteln mit gelösten Polymeren durch sehr niedrige Viskositäten aus. Da es so möglich ist, Mittel mit den genannten hohen Anteilen an Polymeren zu formulieren, kann die erforderliche Menge an Polymer mit einer vergleichsweise geringen Menge an Wasser auf das Haar aufgebracht werden, so daß bei auf dem Haar verbleibenden Mitteln die Trocknungszeit in einem akzeptablen Bereich bleibt.

Insbesondere die Wiederauswaschbarkeit der Haarbehandlungsmittel wird dadurch deutlich erhöht, daß diese noch zusätzlich wasserlösliche Polymere enthalten. Der Begriff "wasserlöslich" ist dabei so zu verstehen, daß die im erfindungsgemäßen Haarbehandlungsmittel enthaltene Menge dieses Polymeren bei Raumtemperatur wasserlöslich ist. In der Regel sind diese, unten im einzelnen aufgeführten, Polymeren zu deutlich mehr als 1 Gew.-% in Wasser löslich.

Bevorzugt sind die wasserlöslichen Polymere in den erfindungsgemäß verwendeten Haarbehandlungsmitteln in Mengen von 0,05 bis 5 Gew.-%, bezogen auf das gesamte Mittel enthalten. In vielen Fällen sind bereits Mengen von ca. 1 % Gew.-% oder weniger, bezogen auf das gesamte Mittel, ausreichend.

Es ist ebenfalls bevorzugt, daß wasserlösliche Polymere in Mengen von 1 bis 20 Gew.-%, bezogen auf das dispergierte, unlösliche Polymere, einzusetzen.

Dieses wasserlösliche Polymere kann auch als Stabilisierungsmittel bei der Emulsionspolymerisation der unlöslichen Polymeren dienen, also bereits bei der Herstellung des unlöslichen Polymeren zugegeben werden. Üblicherweise wird es aber erst bei der Formulierung des Haarbehandlungsmittels zugemischt.

Erfindungsgemäß bevorzugte wasserlösliche Polymere sind nichtionogen, insbesondere, wenn sie erst bei der Formulierung des Haarbehandlungsmittels zugesetzt werden. Geeignete nichtionogene Polymere sind beispielsweise:

- Polyvinylpyrrolidone, wie sie beispielsweise unter der Bezeichnung Luviskol[R] (BASF) vertrieben werden.

- Vinylpyrrolidon/Vinylacetat-Copolymere, wie sie beispielsweise unter dem Warenzeichen Luviskol[R] (BASF) vertrieben werden. Luviskol[R] VA 64 und Luviskol[R] VA 73 sind bevorzugte nichtionogene Polymere.

- Celluloseether, wie Hydroxypropylcellulose, Hydroxyethylcellulose und Methylhydroxypropylcellulose, wie sie beispielsweise unter den Warenzeichen Culminal[R] und Benecel[R] (AQUALON) vertrieben werden.

Geeignete amphotere Polymere sind beispielsweise die unter den Bezeichnungen Amphomer[R] und Amphomer[R] LV-71 (DELFT NATIONAL) erhältlichen Octylacrylamid/Methylmethacrylat/tert. Butylaminoethylmethacrylat/2-Hydroxypropylmethacrylat-Copolymere, deren Verwendung aber in der Regel auf neutral oder alkalisch eingestellte Mittel beschränkt ist.

Geeignete zwitterionische Polymere sind beispielsweise die in den deutschen Patentanmeldungen DE 39 29 973, DE 21 50 557, DE 28 17 369 und DE 37 08 451 offenbarten Polymerisate. Acrylamidopropyltimethylammoniumchlorid/Acrylsäure- bzw. -Methacrylsäure-Copolymerisate und deren Alkali- und Ammoniumsalze sind besonders bevorzugte zwitterionische Polymere. Weiterhin geeignete zwitterionische Polymere sind Methacroylethylbetain/Methacrylat-Copolymere, die unter der Bezeichnung Amersette[R] (AMERCHOL) im Handel erhältlich sind.

Erfindungsgemäß geeignete anionische Polymere sind u. a.:

- Vinylacetat/Crotonsäure-Copolymere, wie sie beispielsweise unter den Bezeichnungen Resyn[R] (NATIONAL STARCH), Luviset[R] (BASF) und Gafset[R] (GAF) im Handel sind.

- Vinylpyrrolidon/Vinylacrylat-Copolymere, erhältlich beispielsweise unter dem Warenzeichen Luviflex[R] (BASF). Ein bevorzugtes Polymer ist das unter der Bezeichnung Luviflex[R] VBM-35 (BASF) erhältliche Vinylpyrrolidon/Acrylat-Terpolymere.

- Acrylsäure/Ethylacrylat/N-tert.Butylacrylamid-Terpolymere, die beispielsweise unter der Bezeichnung Ultrahold[R] strong (BASF) vertrieben werden.

Zur Erzielung der erfindungsgemäßen Vorteile ist auch die Verwendung anionischer Polymerer in der Regel auf neutral oder insbesondere alkalisch eingestellte Lösungen begrenzt.

Kationische Polymere sind nur in Ausnahmefällen als lösliche Polymere geeignet.

In den Fällen, in denen das wasserunlösliche Polymere ionische Gruppen enthält, hat es sich als zweckmäßig erwiesen, wenn das wasserlösliche Polymere nichtionogen oder von gleicher Ionogenität ist.

In einigen Fällen hat sich die Kombination dispergierter anionischer Polymerer und gelöster anionischer Polymerer als besonders vorteilhaft erwiesen. Weiterhin ist es in vielen Fällen vorzuziehen, das lösliche Polymer erst der ansonsten fertig zusammengestellten Zubereitung zuzugeben.

Die weiteren Bestandteile der erfindungsgemäß verwendeten Haarbehandlungsmittel sind von der Art des Haarbehandlungsmittels abhängig. Prinzipiell umfassen die erfindungsgemäß verwendeten Formulierungen alle bekannten Arten von Haarbehandlungsmitteln wie z. B. Haarfestiger, Haarsprays, Fönwellen, Haarshampoos, Haarspülungen, Haarkonditioniermittel, Haarkuren, Dauerwellmittel, und Haarfärbemittel.

Bevorzugte erfindungsgemäß verwendete Haarbehandlungsmittel sind solche, die nach der Anwendung auf dem Haar verbleiben. Dies sind inbesondere Haarfestiger, Haarsprays und Fönwellen. Solche Mittel können mit Hilfe eines Treibmittels auch als Schaumaerosol formuliert sein.

Weitere Bestandteile der erfindungsgemäßen Mittel können beispielsweise sein:

- anionische Tenside, wie beispielsweise Fettalkylsulfate und -ethersulfate,
- kationische Tenside, wie beispielsweise quartäre Ammoniumverbindungen,
- zwitterionische Tenside, wie beispielsweise Betaine,
- ampholytische Tenside,
- nichtionogene Tenside, wie beispielsweise Alkylpolyglycoside und ethoxylierte Fettalkohole,
- Strukturanten wie Glucose und Maleinsäure,
- haarkonditionierende Verbindungen wie Phospholipide, beispielsweise Sojalecithin, Ei-Lecithin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes,
- Wirkstoffe wie Panthenol, Allantoin, Pyrrolidoncarbonsäuren und deren Salze, Pflanzenextrakte und Vitamine,
- Lichtschutzmittel,
- Konsistenzgeber wie Zuckerester, Polyolester oder Polyolalkylether,
- Fette und Wachse, wie Walrat, Bienenwachs, Montanwachs, Paraffine und Fettalkohole,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,

- Trübungsmittel wie Latex,
- Perlglanzmittel wie Ethylenglykolmono- und -distearat,
- Treibmittel wie Propan-Butan-Gemische, $N_2O$, Dimethylether, $CO_2$ und Luft sowie
- Antioxidantien,
- direktziehende Farbstoffe,
- sogenannte Kuppler- und Entwicklerkomponenten als Oxidationsfarbstoffvorprodukte,
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat.

Die folgenden Beispiele sollen die Erfindung näher erläutern.

## Beispiele

### 1. Herstellungsbeispiele

1.1 Polymerdispersion 1

In ein Reaktionsgefäß mit Rührer, Heizung, Kühlung, Rückflußkühler, Thermometer und rührbarem Dosiergefäß wurden 445 g entionisiertes Wasser, 134 G-Cryl$^R$ 5000 (wasserlösliches Vinylaromat-Acrylsäure-Copolymeres mit einer Glasübergangstemperatur von ca. 100 °C (HENKEL)) und anschließend portionsweise 30 g einer 25%igen wäßrigen Ammoniaklösung gegeben. Sodann wurde bei Raumtemperatur bis zur Bildung einer homogenen opaken Lösung gerührt. Nach mehrmaligem Evakuieren und Befüllen des Reaktionsgefäßes mit Reinststickstoff wurden 10 g Dowfax 2A1 (4-Dodecyldiphenyletherdisulfonat-Dinatriumsatz, 45 % Aktivsubstanz in Wasser (DOW)) zugegeben und der Ansatz auf 85 °C erhizt. Dann wurden 3,5 g Ammoniumperoxidisulfat, gelöst in 18 g entionisiertem Wasser, zugesetzt und anschließend über einen Zeitraum von 75 Minuten eine Mischung, bestehend aus 185 g Methylmethacrylat, 134 g Butylacrylat und 15 g Dehydrophen$^R$ 65 (Nonylphenol + 6,5 Ethylenoxid (HENKEL)), gleichmäßig zudosiert. Dabei wurde die Temperatur des Ansatzes durch Außenkühlung auf Werten knapp unterhalb von +88 °C gehalten. Zu dem Zeitpunkt, als 80 % der Monomerenemulsion zudosiert waren, wurden noch weitere 0,5 g Ammoniumperoxidisulfat, gelöst in 15 g entionisiertem Wasser, zugesetzt. Nach Beendigung des Monomerenzulaufs wurde der Ansatz noch weitere 60 Minuten bei +85 °C gerührt. Anschließend wurde auf unter +40 °C abgekühlt, die entstandene Dispersion mit 10 g Polypropylenglykol (Molmasse ca. 1000; (DOW)) versetzt und durch einen Perlonsiebbeutel mit 80 μm Maschenweite filtriert.

Die koagulatfreie milchige Polymerdispersion hatte eine Viskosität von ca. 3000 mPas; die Glastemperatur des dispergierten Polymeren lag bei 20 °C.

1.2 Polymerdispersion 2

In ein Reaktionsgefäß mit Rührer, Heizung, Kühlung, Rückflußkühler, Thermometer und rührbarem Dosiergefäß wurden 445 g entionisiertes Wasser, 134 G-Cryl$^R$- 5000 (wasserlösliches Vinylaromat-Acrylsäure-Copolymeres mit einer Glasübergangstemperatur von ca. 100 °C (HENKEL)) und anschließend portionsweise 30 g einer 25%igen wäßrigen Ammoniaklösung gegeben. Sodann wurde bei Raumtemperatur bis zur Bildung einer homogenen opaken Lösung gerührt. Nach mehrmaligem Evakuieren und Befüllen des Reaktionsgefäßes mit Reinststickstoff wurden 10 g Dowfax 2A1 (4-Dodecyldiphenyletherdisulfonat-Dinatriumsalz, 45 % Aktivsubstanz in Wasser (DOW)) zugegeben und der Ansatz auf 85 °C erhizt. Dann wurden 3,5 g Ammoniumperoxidisulfat, gelöst in 18 g entionisiertem Wasser, zugesetzt und anschließend über einen Zeitraum von 75 Minuten eine Mischung, bestehend aus 123 g Methylmethacrylat, 89 g 2-Ethylhexcylacrylat, 62 g Styrol, 45 g Butylacrylat und 15 g Dehydrophen$^R$ 65 (Nonylphenol + 6,5 Ethylenoxid (HENKEL)), gleichmäßig zudosiert. Dabei wurde die Temperatur des Ansatzes durch Außenkühlung auf Werten knapp unterhalb von +88 °C gehalten. Zu dem Zeitpunkt, als 80 % der Monomerenemulsion zudosiert waren, wurden noch weitere 0,5 g Ammoniumperoxidisulfat, gelöst in 15 g entionisiertem Wasser, zugesetzt. Nach Beendigung des Monomerenzulaufs wurde der Ansatz noch weitere 60 Minuten bei +85 °C gerührt. Anschließend wurde auf unter +40 °C abgekühlt, die entstandene Dispersion mit 10 g Polypropylenglykol (Molmasse ca. 1000; (DOW)) versetzt und durch einen Perlonsiebbeutel mit 80 μm Maschenweite filtriert.

Es wurde eine koagulatfreie milchige Polymerdispersion mit 46,9 % Trockenrückstand (gemessen mit einer Mettler-Trocknungswaage LP 15 auf Stufe 10 über 1 Stunde) und einer Brookfield-Viskosität von ca. 1500 mPas (gemessen mit einem Brookfield-Viskosimeter RTV bei 20 °C und 20 UpM mit Spindel 2) erhalten. Der pH-Wert der Dispersion lag bei 8, die Glastemperatur des dispergierten Polymeren bei 20 °C.

1.3 Polymerdispersion 3

In einem Reaktionsgefäß mit Rührer, Heizung, Kühlung, Rückflußkühler, Thermometer und rührbarem Dosiergefäß wurden 396 g entionisiertes Wasser und 1,5 g Disponil$^R$FES 993 (Fettalkoholpolyglykolethersulfat mit einem Aktivsubstanzgehalt von ca. 30 % in Wasser (HENKEL)) auf 82 °C erhitzt.
Dann wurden 0,5 g Kaliumperoxidisulfat, gelöst in 12 g entionisiertem Wasser, hinzugegeben. Innerhalb von zwei Stunden wurde dann gleichmäßig die nachfolgend beschriebene Emulsion zudosiert. Die organische Phase der Emulsion bestand aus 152 g Butylacrylat, 194 g Methylmethacrylat und 10 g Methacrylsäure, die wäßrige Phase bestand aus 150 g entionisiertem Wasser, 18,5 g Disponil$^R$FES 993 und 1 g Kaliumperoxidisulfat; die Emulsion wurde durch Rühren in einem Dosiergefäß hergestellt und durch Weiterrühren stabil gehalten. Während des Zudosierens wurde die Temperatur des Ansatzes durch Außenkühlung auf Werten knapp unterhalb von +85 °C gehalten. Nach beendetem Zulauf wurde der Ansatz weitere 20 Minuten bei +85 °C gerührt. Sodann wurden noch 0,5 g Kaliumperoxidisulfat, gelöst in 12 g entionisiertem Wasser, hinzugegeben und noch weitere 45 Minuten bei +85 °C gerührt. Nach dem Abkühlen auf unter +40 °C wurde die Dispersion mit ca. 5 g 12,5 %-iger Ammoniaklösung bis zu einem pH-Wert von 6,5 neutralisiert und durch einen Perlonsiebbeutel mit 80 μm Maschenweite filtriert.
Es wurde eine dünnflüssige koagulatfreie milchige Polymerdispersion mit 38,3 % Trockenrückstand (gemessen mit einem Mettler IR-Trocknungsgerät auf Stufe 10 über 1 Stunde) und einer mittleren Teilchengröße von 115 nm erhalten. Die Glastemperatur der dispergierten Polymeren betrug ca. 20 °C.

## 2. Anwendungstechnische Untersuchungen

2.1 "Curl Retention"-Test

2.1.1. Meßverfahren

Die Messung wurde an 5 Haarsträhnen (# 6925 der Firma Fischbach und Miller; je 0,5 g Masse und 28 cm Länge) bei 22 °C und 90 % rel. Luftfeuchtigkeit durchgeführt.
Die gewogene, trockene Strähne wurde in die zu untersuchende Polymerdispersion (jeweils 5 Gew.-% Polymer) getaucht, wobei durch mehrmaliges Eintauchen und Herausnehmen eine gleichmäßige Verteilung sichergestellt ist. Die überschüssige Polymerdispersion wurde zwischen Daumen und Zeigefinger abgestreift, und die Strähne auf eine Gewichtszunahme von ca. 0,3 g (bezogen auf die unbehandelte Strähne) eingestellt. Sodann wurde die feuchte Strähne auf Spiralwickel (0/ ca. 7 mm) aufgewickelt und über Nacht getrocknet. Danch wurde die Locke abgewickelt und 24 Stunden lang bei 22 °C und 90 % rel. Luftfeuchtigkeit ausgehängt.
Der "Curl Retention"-Wert ergibt sich dann aus der Beziehung:

$$c_r = (1 - l_{24})/(1 - l_o) * 100.$$

Dabei bedeuten:

l: Länge der Haarsträhne zwischen 1. und 5. Wellenmitte im gezogenen Zustand
$l_{24}$: Abstand zwischen 1. und 5. Wellenmitte nach 24stündigem Aushängen
$l_o$: Abstand zwischen 1. und 5. Wellenmitte unmittelbar nach der Entfernung des Spiralwickels.

Mit den Dispersionen gemäß Beispielen 1.2 und 1.3 wurden Curl-Retention-Werte von 58 bzw. 60 % erhalten.
Ein gutes Curl-Retention-Niveau wird auch bei Variation dieser Dispersionen im angegebenen Tg-Bereich erhalten. Bei sehr niedrigen Tg-Werten sind die Filme zu weich, bei sehr hohen Tg-Werten zu spröde; in beiden Fällen fallen auch die Curl-Retention-Werte deutlich ab.

2.2.2. Auswaschbarkeit

Haarsträhnen (# 6923 der Firma Fischbach und Miller, ca. 2 g, ca. 15 cm lang), die mit den zu untersuchenden Zubereitungen besprüht wurden (jeweils 1 g Spray, enthaltend 20 Gew.-% Polymer) wurden nach 24 Stunden mit einer Tensidlösung (10 % Texapon$^R$N 28 (Natriumlaurylethersulfat, 28 % Aktivsubstanz in Wasser (HENKEL)) in Wasser gewaschen, getrocknet und visuell auf Rückstände geprüft.
Dispersionen gemäß Beispiel 1.1 und 1.2 waren gut auswaschbar.
Eine Dispersion gemäß Beispiel 1.3 wies noch Rückstände auf. Durch Zugabe von 1 % Luviskol$^R$ VA 64 (Vinylacetat-Vinylpyrrolidon-Copolymer (BASF)) entstand dagegen eine Dispersion, die rückstandsfrei auswaschbar ist.

2.2.3. Versprühbarkeit

Alle Dispersionen waren auch bei hohen Polymerkonzentrationen (20 %) gut versprühbar. Die Düsen verstopften auch nach längerem Verbrauch nicht.

**3. Anwendungsrezepturen**

(alle Angaben sind Gewichtsteile)

3.1 Pumpspray

| | |
|---|---|
| Panthenol | 0,5 |
| Parfümöl | 0,2 |
| Luviskol[R]VA64[1] | 1,0 |
| 20%ige Dispersion des Polymeren gemäß Beispiel 1.1 | ad 100 |

[1] Vinylacetat-Vinylpyrrolidon-Copolymer (BASF)

3.2 Pumpspray

| | |
|---|---|
| Panthenol | 0,5 |
| Parfümöl | 0,2 |
| 20%ige Dispersion des Polymeren gemäß Beispiel 1.2 | ad 100 |

**Patentansprüche**

**1.**   Verwendung eines unlöslichen Polymeren mit einer Glasübergangstemperatur zwischen -20 und +70°C, bestehend im wesentlichen aus Monomeren, ausgewählt aus der Gruppe, die Ester der Acrylsäure, Ester der Methacrylsäure und Styrol umfaßt, in Haarbehandlungsmitteln zur Behandlung von Haaren mit der Maßgabe, daß die Haarbehandlungsmittel, enthaltend übliche kosmetische Bestandteile, in Form einer wäßrigen Dispersion dieses Polymeren vorliegen, weiterhin ein wasserlösliches Polymer enthalten und Wasser als einziges Lösungsmittel enthalten.

**2.**   Verwendung nach Anspruch 1, **dadurch gekennzeichnet, daß** das Polymer zu mindestens 90 Gew.-% aus Monomeren der Gruppe besteht, die von den Estern der Acryl- oder Methacrylsäure mit $C_{1-22}$-Alkoholen und Styrol gebildet wird, mit der Maßgabe, daß der Gehalt an Styroleinheiten nicht mehr als 70 Gew.-%, bezogen auf das gesamte Polymer, beträgt.

**3.**   Verwendung nach Anspruch 1 und 2, **dadurch gekennzeichnet, daß** die Ester der Acryl- und Methacrylsäure ausgewählt sind aus den Estern mit $C_{1-6}$-, insbesondere $C_{1-4}$-, Alkoholen.

**4.**   Verwendung nach einem der Ansprüche 1 bis 3, **dadurch gekennzeichnet, daß** das Polymer mindestens zwei verschiedene Monomere, ausgewählt aus Estern der Acryl- oder Methacrylsäure oder Styrol, enthält.

**5.**   Verwendung nach einem der Ansprüche 1 bis 4, **dadurch gekennzeichnet, daß** das Polymer in einer Menge von 0,1 bis 30 Gew.-%, insbesondere 1 bis 20 Gew.-%, in dem Haarbehandlungsmittel enthalten ist.

**6.**   Verwendung nach einem der Ansprüche 1 bis 5, **dadurch gekennzeichnet, daß** das wasserlösliche Polymer in Mengen von 0,05 bis 5 Gew.-%, bezogen auf das gesamte Mittel, enthalten ist.

**7.**   Verwendung nach einem der Ansprüche 1 bis 6, **dadurch gekennzeichnet, daß** das wasserlösliche Polymer nichtionogen ist.

**Claims**

1. The use of an insoluble polymer with a glass transition temperature of -20 to +70°C consisting essentially of monomers selected from the group comprising esters of acrylic acid, esters of methacrylic acid and styrene, in hair treatment compositions for treating hair, with the proviso that the hair treatment compositions containing typical cosmetic ingredients are present in the form of an aqueous dispersion of the polymer, additionally contain a water-soluble polymer and contain water as sole solvent.

2. The use claimed in claim 1, **characterized in that** at least 90% by weight of the polymer consists of monomers from the group consisting of esters of acrylic or methacrylic acid with $C_{1-22}$ alcohols and styrene, with the proviso that the content of styrene units is no more than 70% by weight, based on the polymer as a whole.

3. The use claimed in claims 1 and 2, **characterized in that** the esters of acrylic and methacrylic acid are selected from the esters with $C_{1-6}$ and more particularly $C_{1-4}$ alcohols.

4. The use claimed in any of claims 1 to 3, **characterized in that** the polymer contains at least two different monomers selected from the esters of acrylic or methacrylic acid or styrene.

5. The use claimed in any of claims 1 to 4, **characterized in that** the polymer is present in the hair treatment composition in a quantity of 0.1 to 30% by weight and more particularly 1 to 20% by weight.

6. The use claimed in any of claims 1 to 5, **characterized in that** the water-soluble polymer is present in quantities of 0.05 to 5% by weight, based on the composition as a whole.

7. The use claimed in any of claims 1 to 6, **characterized in that** the water-soluble polymer is nonionic.

**Revendications**

1. Utilisation d'un polymère insoluble ayant une température de transition vitreuse comprise entre - 20 et + 70 °C, consistant pour l'essentiel en des monomères choisis dans le groupe qui comprend les esters de l'acide acrylique, les esters de l'acide méthacrylique, et le styrène, dans des produits de traitement capillaire avec la restriction que les produits de traitement capillaire contenant des constituants cosmétiques usuels, se présentent sous forme d'une dispersion aqueuse de ce polymère, renferment en outre un polymère soluble dans l'eau et contiennent de l'eau comme unique solvant.

2. Utilisation selon la revendication 1,
   **caractérisée en ce que**
   le polymère consiste pour au moins 90 % en poids en des monomères du groupe qui est formé par les esters d'acide acrylique ou méthacrylique avec les alcools en $C_1$-$C_{22}$ et le styrène, avec la restriction que la teneur en éléments styrène ne s'élève pas à plus de 70 % en poids rapporté à la totalité du polymère.

3. Utilisation selon l'une des revendications 1 et 2,
   **caractérisée en ce que**
   les esters de l'acide acrylique et de l'acide méthacrylique sont choisis parmi les esters avec des alcools en $C_1$-$C_6$, en particulier des alcools en $C_1$-$C_4$.

4. Utilisation selon l'une quelconque des revendications 1 à 3,
   **caractérisée en ce que**
   le polymère contient au moins deux polymères différents choisis parmi les esters d'acide acrylique et d'acide méthacrylique ou le styrène.

5. Utilisation selon l'une quelconque des revendications 1 à 4,
   **caractérisée en ce que**
   le polymère est contenu en une quantité allant de 0,1 à 30 % en poids, en particulier de 1 à 20 % en poids, dans le produit de traitement capillaire.

6. Utilisation selon l'une quelconque des revendications 1 à 5,
**caractérisée en ce que**
le polymère soluble dans l'eau est contenu en une quantité allant de 0,05 à 5 % en poids, rapporté à la totalité du produit.

7. Utilisation selon l'une quelconque des revendications 1 à 6,
**caractérisée en ce que**
le polymère soluble dans l'eau est non ionogène.